# EUROPEAN PATENT SPECIFICATION

(11) **EP 3 603 634 B1**
(45) Date of publication and mention of the grant of the patent: **12.02.2025**
(21) Application number: 19180535.7
(22) Date of filing: 17.05.2005
(51) Int. Cl.: A61K 31/40, A61K 31/4704, A61P 11/00, A61K 45/06

(54) **PHARMACEUTICAL COMPOSITION CONTAINING GLYCOPYRROLATE AND A BETA2 ADRENOCEPTOR AGONIST**
PHARMAZEUTISCHE ZUSAMMENSETZUNG ENTHALTEND GLYKOPYRROLAT UND EINEN BETA2-ADRENOCEPTOR ANTAGONISTEN
COMPOSITION PHARMACEUTIQUE COMPRENANT DU GLYCOPYRROLATE ET UN AGONISTE DU RÉCEPTEUR BETA2 ADRÉNERGIQUE

(30) Priority: 18.05.2004 GB 0411056
(43) Date of publication of application: 05.02.2020
(62) Divisional of application: 10164976.2
(73) Proprietor: Novartis AG, 4056 Basel (CH); Novartis Pharma GmbH, 1230 Wien (AT)
(72) Inventor: Collingwood, Stephen Paul, Sussex, Horsham RH12 5 AB (GB)
(74) Representative: Carpmaels & Ransford LLP

(56) References cited:
- WO-A1-00/75114
- WO-A2-01/76575
- US-A1- 2004 002 548
- CYDULKA R K ET AL: "EFFECTS OF COMBINED TREATMENT WITH GLYCOPYRROLATE AND ALBUTEROL IN ACUTE EXACERBATION OF CHRONIC OBSTRUCTIVE PULMONARY DISEASE", ANNALS OF EMERGENCY MEDICINE, LANSING, MI, US, vol. 25, April 1995 (1995-04-01), pages 470 - 473, XP000979833, ISSN: 0196-0644
- TZELEPIS G ET AL: "COMPARISON OF NEBULIZED GLYCOPYRROLATE AND METAPROTERENOL IN CHRONIC OBSTRUCTRIVE PULMONARY DISEASE", EUROPEAN RESPIRATORY JOURNAL, MUNKSGAARD INTERNATIONAL PUBLISHERS, COPENHAGEN, DK, vol. 9, no. 8, 1996, pages 100 - 103, XP000979863, ISSN: 0903-1936
- CALVO G M ET AL: "IS IT USEFUL TO ADD AN ANTICHOLINERGIC TREATMENT TO BETA2-ADRENERGIC MEDICATION IN ACUTE ASTHMA ATTACK", JOURNAL OF INVESTIGATIONAL ALLERGOLOGY AND CLINICAL IMMUNOLOGY, BARCELONA, ES, vol. 8, no. 1, January 1998 (1998-01-01), pages 30 - 34, XP009018610, ISSN: 1018-9068
- NOORD VAN J A ET AL: "LONG-TERM TREATMENT OF CHRONIC OBSTRUCTIVE PULMONARY DISEASE WITH SALMETEROL AND THE ADDITIVE EFFECT OF IPRATROPIUM", EUROPEAN RESPIRATORY JOURNAL, MUNKSGAARD INTERNATIONAL PUBLISHERS, COPENHAGEN, DK, vol. 15, no. 5, May 2000 (2000-05-01), pages 878 - 885, XP001021107, ISSN: 0903-1936
- WESSELING G ET AL: "COMPARISON OF THE EFFECTS OF ANTICHOLINERGIC AND BETA2-AGONIST AND COMBINATION THERAPY ON RESPIRATORY IMPEDANCE IN COPD", CHEST, THE COLLEGE, CHICAGO, IL, US, vol. 101, no. 1, 1992, pages 166 - 173, XP000913861, ISSN: 0012-3692
- BACULARD A: "PLACE DU BRONCHODUAL DANS LE TRAITEMENT DE FOND DE L'ASTHME DE L'ENFANT BRONCHODUAL IN THE LONG-TERM TREATMENT OF CHILDREN WITH ASTHMA", ARCHIVES DE PEDIATRIE, ELSEVIER, PARIS, FR, vol. 2, no. SUPPL 2, 1995, pages 149S - 153S, XP009010409, ISSN: 0929-693X
- BRIGGS D D ET AL: "Long-term pharmacologicmanagement of patients with chronic obstructive pulmonary disease", CLINICAL CORNERSTONE, EXCERPTA MEDICA,, GB, vol. 6, no. 2, 2004, pages S17 - S28, XP004825197, ISSN: 1098-3597
- CAZZOLA ET AL: "Incremental Benefit of Adding Oxitropium Bromide to Formoterol in Patients with Stable COPD", PULMONARY PHARMACOLOGY AND THERAPEUTICS, ACADEMIC PRESS, NEW YORK, NY, US, vol. 12, 1999, pages 267 - 271, XP002210290, ISSN: 1094-5539

## Description

This invention relates to organic compounds and their use as pharmaceuticals, in particular for the treatment of inflammatory or obstructive airways diseases.

Cydulka et al, Annals of Emergency Medicine, volume 25, April 1995, pages 470-473 discloses treating patients that have acute exacerbation of chronic obstructive pulmonary disease with nebulised glycopyrrolate and nebulised albuterol.

Tzelepis et al, European Respiratory Journal, 1996, 9, pages 100-103 discloses treating patients that have chronic obstructive pulmonary disease with nebulised glycopyrrolate and nebulised metaproterenol.

The invention is defined by the appended claims. In one aspect, the present invention provides the use of
(A) glycopyrrolate; and
(B) a compound of formula I 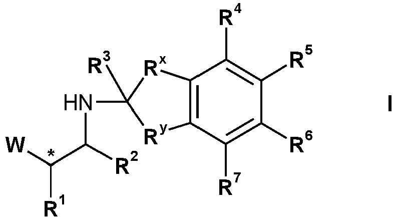
   in free or salt or solvate form,
   wherein W is a group of formula 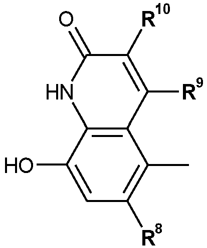
   R⁸, R⁹ and R¹⁰ are each H, R¹ is OH, R² and R³ are each H and
   R^{x} and R^{y} are both -CH₂-, and R⁴ and R⁷ are each H and R⁵and R⁶ are each CH₃CH₂-; in the preparation of a medicament for combination therapy by simultaneous administration of (A) and (B) in the treatment of an inflammatory or obstructive airways disease, wherein the administration is by inhalation, and wherein (A) and (B) are present in admixture in inhalable form as a dry powder comprising finely divided (A) and (B) optionally together with the least one particulate pharmaceutically acceptable carrier.

Preferably the molar ratio of (A) to (B) is from 100:1 to 1:300, for example 50:1 to 1:100, especially from 10:1 to 1:20, and more especially from 3:1 to 1:7.

(A) Glycopyrrolate is a known antimuscarinic agent. More specifically it inhibits acetyl choline binding to M3 muscarinic receptors thereby inhibiting bronchoconstriction.

Glycopyrrolate is a quaternary ammonium salt. Suitable counter ions are pharmaceutically acceptable counter ions including, for example, fluoride, chloride, bromide, iodide, nitrate, sulfate, phosphate, formate, acetate, trifluoroacetate, propionate, butyrate, lactate, citrate, tartrate, malate, maleate, succinate, benzoate, p-chlorobenzoate, diphenyl-acetate or triphenylacetate, o-hydroxybenzoate, p-hydroxybenzoate, 1-hydroxynaphthalene-2-carboxylate, 3-hydroxynaphthalene-2-carboxylate, methanesulfonate and benzenesulfonate.

Its bromide salt, namely 3-[(cyclopentyl-hydroxyphenylacetyl)oxy]-1,1-dimethylpyrrolidinium bromide, has the following structural formula 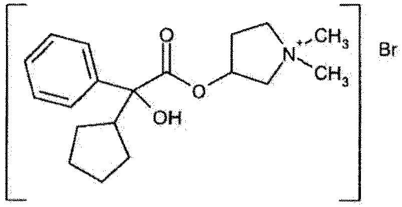 and can be prepared using the procedures described in United States patent US 2956062.

Glycopyrrolate has two stereogenic centres and hence exists in four isomeric forms, namely (3R,2'R)-, (3S,2'R)-, (3R,2'S)- and (3S,2'S)-3-[(cyclopentyl-hydroxyphenylacetyl)oxy]-1,1-dimethylpyrrolidinium bromide, as described in United States patent specifications US 6307060 and US 6,613,795. The present invention embraces using one or more of these isomeric forms, especially the 3S,2'R isomer, the 3R,2'R isomer or the 2S,3'R isomer, thus including single enantiomers, or racemates, especially the (3S,2'R/2S,3'R) racemate.

(B) is a compound of formula I as hereinbefore defined. Compounds of this formula possess beta-2 adrenoceptor agonist activity. They commonly have a rapid onset of action and have a prolonged stimulating action on the β2-adrenoceptor, for example up 24 hours or longer.

Compounds of formula I in free or salt or solvate form may be prepared by using the procedures described in international patent applications WO 2000/075114, WO 2003/076387, WO 2004/076422 or WO 2004/087668.

Compounds of formula I in free form may be converted into salt form, and vice versa, in a conventional manner. The compounds in free or salt form can be obtained in the form of hydrates or solvates containing a solvent used for crystallisation. Compounds of formula can be recovered from reaction mixtures and purified in a conventional manner. Isomers, such as enantiomers, may be obtained in a conventional manner, e.g. by fractional crystallisation or asymmetric synthesis from correspondingly asymmetrically substituted, e.g. optically active, starting materials.

Pharmaceutically acceptable acid addition salts of the compounds of formula I include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxy-benzoic acid, 1-hydroxynaphthalene-2-carboxylic acid or 3-hydroxynaphthalene-2-carboxylic acid, and sulfonic acids such as methanesulfonic acid or benzenesulfonic acid.

These salts may be prepared by known salt-forming procedures. Pharmaceutically acceptable solvates are generally hydrates. Isomers, such as enantiomers, may be obtained in a conventional manner, e.g. by fractional crystallisation or asymmetric synthesis from correspondingly asymmetrically substituted, e.g. optically active, starting materials.

The medicament of the present invention may additionally contain one or more co-therapeutic agents such as anti-inflammatory, bronchodilatory, antihistamine, decongestant or anti-tussive drug substances, particularly in the treatment of obstructive or inflammatory airways diseases such as those mentioned hereinbefore, for example as potentiators of therapeutic activity of such drugs or as a means of reducing required dosaging or potential side effects of such drugs.

Co-therapeutic agents include steroids, A_{2A} agonists, A_{2B} antagonists, antihistamines, caspase inhibitors, LTB4 antagonists, LTD4 antagonists, PDE4 inhibitors, mucolytics, matrix metalloproteinase inhibitors (MMPi's), leukotrienes, antibiotics, anti neoplastics, peptides, vaccines, nicotine, elastase inhibitors and sodium cromoglycate.

Such anti-inflammatory drugs include steroids, for example glucocorticosteroids such as budesonide, beclamethasone dipropionate, fluticasone propionate, ciclesonide or mometasone furoate, or steroids described in WO 02/88167, WO 02/12266, WO 02/100879, WO 02/00679 (especially those of Examples 3, 11, 14, 17, 19, 26, 34, 37, 39, 51, 60, 67, 72, 73, 90, 99 and 101), WO 03/35668, WO 03/48181, WO 03/62259, WO 03/64445, WO 03/72592, WO 04/39827 and WO 04/66920, and non-steroidal glucocorticoid receptor agonists, such as those described in DE 10261874, WO 00/00531, WO 02/10143, WO 03/82280, WO 03/82787, WO 03/86294, WO 03/104195, WO 03/101932, WO 04/05229, WO 04/18429, WO 04/19935, WO 04/26248, WO 0505452. Suitable A_{2A} agonists include those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99167264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618 and WO 04/046083. Suitable A_{2B} antagonists include those described in WO 03/042214 and WO 02/42298. Suitable antihistamine drug substances include cetirizine hydrochloride, acetaminophen, clemastine fumarates promethazine, loratidine, desloratidine, diphenhydramine and fexofenadine hydrochloride, activastine, astemizole, azelastine, ebastine, epinastine, mizolastine and tefenadine as well as those disclosed in WO 03/099807, WO 04/026841, JP 2004107299. Suitable caspase inhibitors, including interleukin- I P converting enzyme (ICE) inhibitors, include those that are disclosed in Canadian patent specification 2109646, EP519748, EP 547 699, EP 590 650, EP 628550, EP 644197, EP 644198, WO 93/05071, WO 93/14777, WO 93/16710, WO 94/00154, WO 94/03480, WO 94/21673, WO 95/05152, WO 95/35308, WO 97/22618, WO 97/22619, WO 98/41232, WO 99/06367, WO 99/65451, WO 01/119373, US 54-11985, US 5416013, US 5430128, US 5434248, US 5565430, US 5585357, US 5656627, US 5677283, US 6054487, US 6531474, US 20030096737, GB 2,278,276 as well as those disclosed in international patent applications WO 98/10778, WO 98/11109, WO 98/11129 and WO 03/32918. Suitable LTB4 antagonists include LY293111, CGS025019C, CP-195543, SC-53228, BIIL 284, ONO 4057, SB 209247 and those described in US 5451700 and WO 04/108720. Suitable LTD4 antagonists include montelukast and zafirlukast. Suitable PDE4 inhibitors PDE4 inhibitors such as cilomilast (Ariflo^{®} GlaxoSmithKline), Roflumilast (Byk Gulden),V-11294A (Napp), BAYI 9-8004 (Bayer), SCH-351591 (Schering-Plough), Arofylline (Almirall Prodesfarma), PDI 89659 / PD168787 (Park-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), GRC 3886 (Glenmark), and those described in WO 92/19594, WO 93/19749, WO 93/19750, WO 93/19751, WO 98/18796, WO 99/16766, WO 01/13953, WO 03/104204, WO 03/104205, WO 03/39544, WO 04/000814, WO 04/000839 and WO 04/005258, WO 04018450, WO 04/018451, WO 04/018457, WO04/018465, WO 04/018431, WO 04/018449, WO 04/018450, WO 04/018451, WO 04/018457, WO 04/018465, WO 04/019944, WO 04/019945, WO 04/045607, WO 4/037805, WO 04/063197, WO 04/103998 and WO 04/111044.

While (A) glycopyrrolate is an M3 antagonist, the medicament of the present invention optionally includes one or more other M3 antagonists such as ipratropium bromide, oxitropium bromide, tiotropium salt, CHF 4226 (Chiesi), or those described in WO 02/51841, WO 02/53564, WO 03/00840, WO 03/87094, WO 04/05285, WO 02/00652, WO 03/53966, EP 424021, US 5171744, US 3714357, WO 03/33495, WO 04/018422 or WO 05/003090.

While (B) are beta-2 adrenoceptor agonists, the medicament of the present invention optionally includes one or more other beta-2 adrenoceptor agonists such as albuterol (salbutamol), metaproterenol, terbutaline, salmeterol fenoterol, procaterol, and especially, formoterol, carmoterol and pharmaceutically acceptable salts thereof, compounds (in free or salt or solvate form) of formula 1 of WO 04/087142, or those described in JP 05025045, US 2002/0055651, WO 93/18007, WO 99/64035, WO 01/42193, WO 01/83462, WO 02/066422, wo 02/070490, WO 02/076933, WO 03/24439, WO 03/725395 WO 03/42160, WO 03/91204, WO 03/42164, WO 03/99764, WO 04/11416, WO 04/16578, WO 04/22547, WO 04/32921, WO 04/33412, WO 04/37773, WO 04/37807, WO 04/39762, WO 04/39766, WO 04/45618, WO 04/46083, WO 04/80964, WO 04/108675 or WO 04/108676.

Administration of the medicament as hereinbefore described,
i.e. with (A) and (B) in admixture, is by inhalation, i.e. (A) and (B) are in inhalable form..

The inhalable form is a dry powder, i.e. (A) and (B) are present in a dry powder comprising finely divided (A) and (B) optionally together with at least one particulate pharmaceutically acceptable carrier, which may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran, mannitol or sorbitol. An especially preferred carrier is lactose. The dry powder may be contained as unit doses in capsules of, for example, gelatin or plastic, or in blisters (e.g. of aluminium or plastic), for use in a dry powder inhalation device, which may be a single dose or multiple dose device, preferably in dosage units of (A) and (B) together with the carrier in amounts to bring the total weight of powder per capsule to from 5 mg to 50 mg. Alternatively, the dry powder may be contained in a reservoir in a multi-dose dry powder inhalation device adapted to deliver, for example, 3-25mg of dry powder per actuation.

In the finely divided particulate form of the medicament, the active ingredient may have an average particle diameter of up to about 10 µm, for example 0.1 to 5 µm, preferably 1 to 5 µm. The particulate carrier, where present, generally has a maximum particle diameter up to 300 µm, preferably up to 212 µm, and conveniently has a mean particle diameter of 40 to 100 µm, e.g. 50 to 75 µm. The particle size of the active ingredient, and that of a particulate carrier where present in dry powder compositions, can be reduced to the desired level by conventional methods, for example by grinding in an air-jet mill, ball mill or vibrator mill, sieving, microprecipitation, spray-drying, lyophilisation or controlled crystallisation from conventional solvents or from supercritical media.

The medicament may be a controlled release formulation comprising finely divided particles of (A) and (B) within a hydrophobic matrix material, e.g. comprising magnesium stearate, for example as described in international patent application WO 01/76575.

The inhalable medicament may be administered using an inhalation device suitable for the inhalable form, such devices being well known in the art. Accordingly, the invention also provides a pharmaceutical product comprising a medicament or pharmaceutical composition as hereinbefore described in inhalable form as hereinbefore described in association with one or more inhalation devices. In a further aspect, the invention provides an inhalation device, or a pack of two or more inhalation devices, containing a medicament or pharmaceutical composition as hereinbefore described in inhalable form as hereinbefore described.

Where the inhalable form of the active ingredient is the finely divided particulate form, the inhalation device may be, for example, a dry powder inhalation device adapted to deliver dry powder from a capsule or blister containing a dry powder comprising a dosage unit of (A) and (B) or a multidose dry powder inhalation (MDPI) device adapted to deliver, for example, 3-25 mg of dry powder comprising a dosage unit of (A) and (B) per actuation. The dry powder formulation preferably contains the active ingredients optionally together with a diluent or carrier, such as lactose, of the desired particle size distribution and a compound that helps to protect against product performance deterioration due to moisture e.g. magnesium stearate. Suitable such dry powder inhalation devices are well known. For example, a suitable device for delivery of dry powder in encapsulated form is that described in US 3991761, while a suitable MDPI device is that described in WO 97/20589.

The molar ratio of (A) to (B) may be, in general, from 100:1 to 1:300, for example from 50:1 to 1:100 or from 20:1 to 1:50, preferably from 10:1 to 1:20, more preferably from to 1:10, from 3:1 to 1:7 or from 2:1 to 1:2.

A suitable daily dose of the compound (A), particularly as the bromide salt, for inhalation may be from 10 to 2000 µg, preferably from 60 to 1000 µg, and especially from 80 to 800 µg, e.g. from 20 to 500 µg.

A suitable daily dose of compound (B) for inhalation may be from 10 µg to 2000 µg, for example from 10 to 1500 µg, from 10 to 1000 µg, preferably from 20 to 800 µg, e.g. from 20 to 600 µg or from 20 to 500 µg.

A suitable unit dose of compound (A), particularly as the bromide salt, may be from 10 µg to 2000 µg, preferably from 60 to 1000 µg, especially from 80 to 800 µg, e.g. from 20 to 500 µg.

A suitable unit dose of compound (B) may be from 10 µg to 2000 µg, for example from 10 to 1500 µg, from 10 to 1000 µg, preferably from 20 to 800 µg, e.g. from 20 to 600 µg or from 20 to 500 µg.

These unit doses may be administered once or twice daily in accordance with the daily doses mentioned hereinbefore. A single dose is preferred. The precise unit and daily dose used will of course depend on the condition to be treated, the patient and the efficiency of the inhalation device.

In one preferred embodiment of the invention, the medicament of the invention is a pharmaceutical composition which is a dry powder in a capsule containing a unit dose of (A) and (B), for example for inhalation from a single capsule inhaler, the capsule suitably containing a unit dose of (A) e.g. as hereinbefore described; and a unit dose of (B), e.g. as hereinbefore described, together with a pharmaceutically acceptable carrier as hereinbefore described in an amount to bring the total weight of dry powder per capsule to between 5 mg and 50 mg, for example 5 mg, 10 mg, 15 mg, 20 mg, 25 mg, 30 mg, 35 mg, 40 mg, 45 mg or 50 mg.

In another preferred embodiment of the invention, the medicament of the invention is a pharmaceutical composition which is a dry powder for administration from a reservoir of a multi-dose dry powder inhaler adapted to deliver, for example, 3 mg to 25 mg of powder containing a unit dose of (A) and (B) per actuation, for example, where (A) is in the form of the maleate salt, a powder comprising, by weight, 20 to 2000 parts, for example 60 to 1000 parts, 100 to 500 parts, or 100 to 300 parts of (A); 25 to 800 parts, e.g. 25 to 500 parts, 50 to 400 parts, or 100 to 400 parts of (B); 0.2 to 1 part magnesium stearate, and 2000 to 25000 parts, e.g. 4000 to 15000 parts or 4000 to 10000 parts of a pharmaceutically acceptable carrier as hereinbefore described.

The medicaments of the invention are advantageous in the treatment of inflammatory or obstructive airways disease, exhibiting highly effective bronchodilatory and anti-inflammatory properties. For instance, it is possible using the combination therapy of the invention to reduce the dosages of (A) or (B) required for a given therapeutic effect compared with those required using treatment with either (A) or (B) alone, thereby minimising possibly undesirable side effects. Furthermore, using the combinations of the invention, particularly using compositions containing (A) and (B), medicaments which have a rapid onset of action and a long duration of action may be prepared. Moreover, using such combination therapy, medicaments which result in a significant improvement in lung function may be prepared. In another aspect, using the combination therapy of the invention, medicaments which provide effective control of obstructive or inflammatory airways diseases, or a reduction in exacerbations of such diseases, may be prepared. In a further aspect, using compositions of the invention containing (A) and (B), medicaments which reduce or eliminate the need for treatment with short-acting rescue medicaments such as salbutamol or terbutaline, may be prepared; thus compositions of the invention containing (A) and (B) facilitate the treatment of an obstructive or inflammatory airways disease with a single medicament.

Treatment of inflammatory or obstructive airways diseases in accordance with the invention may be symptomatic or prophylactic treatment. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute/adult lung injury (ALI), adult/acute respiratory distress syndrome (ARDS), cystic fibrosis, chronic obstructive pulmonary, airways or lung disease (COPD, COAD or COLD), including chronic bronchitis and emphysema, bronchiectasis and exacerbation of airways hyperreactivity consequent to other drug therapy, in particular other inhaled drug therapy. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tobacosis and byssinosis.

The invention is illustrated by the following Examples.

### EXAMPLES

Compound A1, namely 3-[(cyclopentyl-hydroxyphenylacetyl)oxy]-1,1-dimethylpyrrolidinium bromide (glycopyrrolate), is commercially available as a racemate or is prepared using the procedures described in United States patent US 2956062.

Compound B1, namely (R)-5-[2-(5,6-diethyl-indan-2-ylamino)-1-hydroxy-ethyl]-8-hydroxy-1H-quinolin-2-one maleate, is prepared using the procedures described in international patent application WO 2000/075114.

### Examples 1-60

Gelatin capsules suitable for use in a capsule inhaler such as that described in US 3991761 and EP 1270034 are prepared, each capsule containing a dry powder obtained by mixing Compound A1 and Compound B1 which have been ground to a mean particle diameter of 1 to 5 µm and lactose monohydrate having a particle diameter below 212 µm, the amounts being as shown in the Table 1 below:

**TABLE 1**

| Example | Compound Al (Parts) | Compound Bl (Parts) | Lactose Parts |
|---|---|---|---|
| 1 | 20 | 100 | 19880 |
| 2 | 40 | 100 | 19860 |
| 3 | 80 | 100 | 19820 |
| 4 | 100 | 100 | 19800 |
| 5 | 120 | 100 | 19780 |
| 6 | 140 | 100 | 19760 |
| 7 | 160 | 100 | 19740 |
| 8 | 180 | 100 | 19720 |
| 9 | 200 | 100 | 19700 |
| 10 | 220 | 100 | 19680 |
| 11 | 240 | 100 | 19660 |
| 12 | 300 | 100 | 19600 |
| 13 | 500 | 100 | 19400 |
| 14 | 1000 | 100 | 18900 |
| 15 | 2000 | 100 | 17900 |
| 16 | 20 | 100 | 24880 |
| 17 | 40 | 100 | 24860 |
| 18 | 80 | 100 | 24820 |
| 19 | 100 | 100 | 24800 |
| 20 | 120 | 100 | 24780 |
| 21 | 140 | 100 | 24760 |
| 22 | 160 | 100 | 34740 |
| 23 | 180 | 100 | 24720 |
| 24 | 200 | 100 | 24700 |
| 25 | 220 | 100 | 24680 |
| 26 | 240 | 100 | 24660 |
| 27 | 300 | 100 | 24600 |
| 28 | 500 | 100 | 24400 |
| 29 | 1000 | 100 | 23900 |
| 30 | 2000 | 100 | 22900 |
| 31 | 20 | 200 | 14780 |
| 32 | 40 | 200 | 14760 |
| 33 | 80 | 200 | 14720 |
| 34 | 100 | 200 | 14700 |
| 35 | 120 | 200 | 14680 |
| 36 | 140 | 200 | 14660 |
| 37 | 160 | 200 | 14640 |
| 38 | 180 | 200 | 14620 |
| 39 | 200 | 200 | 14600 |
| 40 | 220 | 200 | 14580 |
| 41 | 240 | 200 | 14560 |
| 42 | 300 | 200 | 14500 |
| 43 | 500 | 200 | 14300 |
| 44 | 1000 | 200 | 13800 |
| 45 | 2000 | 200 | 12800 |
| 46 | 20 | 200 | 24780 |
| 47 | 40 | 200 | 24760 |
| 48 | 80 | 200 | 24720 |
| 49 | 100 | 200 | 24700 |
| 50 | 120 | 200 | 24680 |
| 51 | 140 | 200 | 24660 |
| 52 | 160 | 200 | 24640 |
| 53 | 180 | 200 | 24620 |
| 54 | 200 | 200 | 24600 |
| 55 | 220 | 200 | 24580 |
| 56 | 240 | 200 | 24560 |
| 57 | 300 | 200 | 24500 |
| 58 | 500 | 200 | 24300 |
| 59 | 1000 | 200 | 23800 |
| 60 | 2000 | 200 | 22800 |

## Claims

1. The use of:
(A) glycopyrrolate; and
(B) a compound of formula I 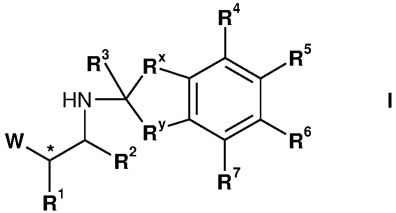
in free or salt or solvate form,
wherein W is a group of formula 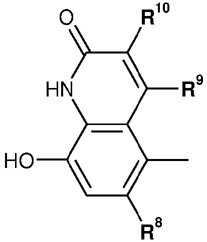
R⁸, R⁹ and R¹⁰ are each H, R¹ is OH, R² and R³ are each H and
R^{x} and R^{y} are both -CH₂-, and R⁴ and R⁷ are each H and R⁵ and R⁶ are each CH₃CH₂-;
in the preparation of a medicament for combination therapy by simultaneous administration of (A) and (B) in the treatment of an inflammatory or obstructive airways disease, wherein the administration is by inhalation,
and wherein (A) and (B) are present in admixture in inhalable form as a dry powder comprising finely divided (A) and (B) optionally together with the least one particulate pharmaceutically acceptable carrier.

2. The use according to claim 1, wherein glycopyrrolate is present as its bromide salt.

3. The use according to claim 1 or 2 wherein glycopyrrolate is a racemate.

4. The use according to claim 1 or 2 wherein glycopyrrolate is a single enantiomer.

5. The use according to claim 4 wherein glycopyrrolate is (3S,2'R )-3-[(cyclopentyl-hydroxyphenylacetyl)oxy]-1,1-dimethylpyrrolidinium bromide or (3R,2'R)-3-[(cyclopentyl-hydroxyphenylacetyl)oxy]-1,1-dimethylpyrrolidinium bromide.

6. The use according to any of claims 1 to 5, in which (B) is a pharmaceutically acceptable addition salt of an inorganic acid or an organic acid of a compound of formula I, selected from hydrofluoric acid, hydrochloric acid, hydrobromic acid, hydroiodic acid, nitric acid, sulfuric acid, phosphoric acid, formic acid, acetic acid, trifluoroacetic acid, propionic acid, butyric acid, lactic acid, citric acid, tartaric acid, malic acid, maleic acid, succinic acid, benzoic acid, p-chlorobenzoic acid, diphenylacetic acid, triphenylacetic acid, o-hydroxybenzoic acid, p-hydroxy-benzoic acid, 1-hydroxynaphthalene-2-carboxylic acid, 3-hydroxynaphthalene-2-carboxylic acid, methanesulfonic acid or benzenesulfonic acid.

7. The use according to any of claims 1 to 3, in which (B) is (R)-5-[2-(5,6-diethyl-indan-2-ylamino)-1-hydroxy-ethyl]-8-hydroxy-1H-quinolin-2-one maleate.

8. The use according to any preceding claim wherein the medicament further contains at least one drug substance which is an anti-inflammatory, a bronchodilator, an antihistamine, a decongestant or an anti-tussive drug substance.

9. The use according to any preceding claim, in which the molar ratio of (A) to (B) is from 100:1 to 1:300.

10. The use according to claim 1, wherein the medicament is a dry powder in a capsule, the capsule containing a unit dose of (A), a unit dose of (B) and a pharmaceutically acceptable carrier in an amount to bring the total weight of dry powder per capsule to between 5 mg and 50 mg.

## Patentansprüche

1. Verwendung von:
(A) Glycopyrrolat und
(B) einer Verbindung der Formel I 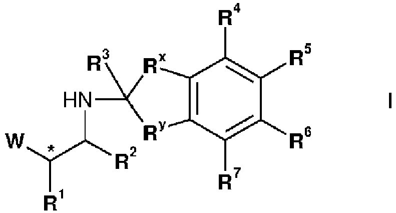
in freier Form oder Salz- oder Solvatform,
wobei W für eine Gruppe der folgenden Formel steht 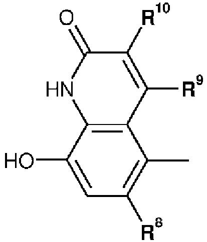
R⁸, R⁹ und R¹⁰ jeweils für H stehen, R¹ für OH steht, R² und R³ jeweils für H stehen und
R^{x} und R^{y} beide für -CH₂- stehen, und R⁴ und R⁷ jeweils für H stehen und R⁵ und R⁶ jeweils für CH₃CH₂- stehen,
bei der Herstellung eines Medikaments zur Kombinationstherapie durch gleichzeitige Verabreichung von (A) und (B) bei der Behandlung einer entzündlichen oder obstruktiven Atemwegserkrankung, wobei die Verabreichung durch Inhalation erfolgt,
und wobei (A) und (B) in Beimischung in inhalierbarer Form als Trockenpulver enthalten sind, das feinteiliges (A) und (B) gegebenenfalls zusammen mit dem mindestens einem teilchenförmigen pharmazeutisch unbedenklichen Träger umfasst.

2. Verwendung nach Anspruch 1, wobei Glycopyrrolat als sein Bromidsalz vorliegt.

3. Verwendung nach Anspruch 1 oder 2, wobei Glycopyrrolat ein Racemat ist.

4. Verwendung nach Anspruch 1 oder 2, wobei Glycopyrrolat ein einzelnes Enantiomer ist.

5. Verwendung nach Anspruch 4, wobei Glycopyrrolat (3S,2'R)-3-[(Cyclopentylhydroxyphenylacetyl)oxy]-1,1-dimethylpyrrolidiniumbromid oder (3R,2'R)-3-[(Cyclopentylhydroxyphenylacetyl)oxy]-1,1-dimethylpyrrolidiniumbromid ist.

6. Verwendung nach einem der Ansprüche 1 bis 5, wobei (B) ein pharmazeutisch unbedenkliches Additionssalz einer anorganischen Säure oder einer organischen Säure einer Verbindung der Formel I ist, ausgewählt aus Fluorwasserstoffsäure, Chlorwasserstoffsäure, Bromwasserstoffsäure, Iodwasserstoffsäure, Salpetersäure, Schwefelsäure, Phosphorsäure, Ameisensäure, Essigsäure, Trifluoressigsäure, Propionsäure, Buttersäure, Milchsäure, Citronensäure, Weinsäure, Äpfelsäure, Maleinsäure, Bernsteinsäure, Benzoesäure, p-Chlorbenzoesäure, Diphenylessigsäure, Triphenylessigsäure, o-Hydroxybenzoesäure, p-Hydroxybenzoesäure, 1-Hydroxynaphthalin-2-carbonsäure, 3-Hydroxynaphthalin-2-carbonsäure, Methansulfonsäure oder Benzolsulfonsäure.

7. Verwendung nach einem der Ansprüche 1 bis 3, wobei (B) (R)-5-[2-(5,6-Diethylindan-2-ylamino)-1-hydroxyethyl]-8-hydroxy-1H-chinolin-2-onmaleat ist.

8. Verwendung nach einem der vorhergehenden Ansprüche, wobei das Medikament weiterhin mindestens einen Arzneistoff enthält, bei dem es sich um eine entzündungshemmende Substanz, einen Bronchodilatator, ein Antihistaminikum, ein Dekongestivum oder ein Antitussivum handelt.

9. Verwendung nach einem der vorangehenden Ansprüche, wobei das Molverhältnis von (A) zu (B) 100:1 bis 1:300 beträgt.

10. Verwendung nach Anspruch 1, wobei das Medikament ein Trockenpulver in einer Kapsel ist, wobei die Kapsel eine Einheitsdosis von (A), eine Einheitsdosis von (B) und einen pharmazeutisch unbedenklichen Träger in solch einer Menge enthält, dass das Gesamtgewicht an Trockenpulver pro Kapsel zwischen 5 mg und 50 mg liegt.

## Revendications

1. Utilisation de :
(A) glycopyrrolate ; et
(B) un composé de formule I 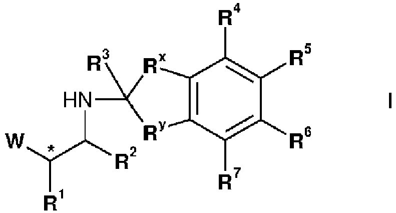
sous forme libre, de sel ou de solvate,
dans laquelle W est un groupe de formule 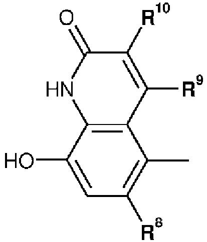
R⁸, R⁹ et R¹⁰ sont chacun H, R¹ est OH, R² et R³ sont chacun H et
R^{x} et R^{y} sont tous deux -CH₂-, et R⁴ et R⁷ sont chacun H et R⁵ et R⁶ sont chacun CH₃CH₂- ;
dans la préparation d'un médicament pour thérapie combinée par administration simultanée de (A) et (B) dans le traitement d'une maladie inflammatoire ou obstructive des voies respiratoires, dans laquelle l'administration est par inhalation,
et dans laquelle (A) et (B) sont présents en mélange sous forme inhalable sous une forme de poudre sèche comprenant (A) et (B) finement divisés éventuellement conjointement avec l'au moins un support pharmaceutiquement acceptable particulaire.

2. Utilisation selon la revendication 1, dans laquelle le glycopyrrolate est présent sous forme de son sel de bromure.

3. Utilisation selon la revendication 1 ou 2, dans laquelle le glycopyrrolate est un racémique.

4. Utilisation selon la revendication 1 ou 2, dans laquelle le glycopyrrolate est un seul énantiomère.

5. Utilisation selon la revendication 4, dans laquelle le glycopyrrolate est le bromure de (3S,2'R)-3-[(cyclopentyl-hydroxyphénylacétyl)oxy]-1,1-diméthylpyrrolidinium ou le bromure de (3R,2'R)-3-[(cyclopentyl-hydroxyphénylacétyl)oxy]-1,1-diméthylpyrrolidinium.

6. Utilisation selon l'une quelconque des revendications 1 à 5, dans laquelle (B) est un sel d'addition pharmaceutiquement acceptable d'un acide inorganique ou d'un acide organique d'un composé de formule I, choisi parmi l'acide fluorhydrique, l'acide chlorhydrique, l'acide bromhydrique, l'acide iodhydrique, l'acide nitrique, l'acide sulfurique, l'acide phosphorique, l'acide formique, l'acide acétique, l'acide trifluoroacétique, l'acide propionique, l'acide butyrique, l'acide lactique, l'acide citrique, l'acide tartrique, l'acide malique, l'acide maléique, l'acide succinique, l'acide benzoïque, l'acide p-chlorobenzoïque, l'acide diphénylacétique, l'acide triphénylacétique, l'acide o-hydroxybenzoïque, l'acide p-hydroxybenzoïque, l'acide 1-hydroxynaphtalène-2-carboxylique, l'acide 3-hydroxynaphtalène-2-carboxylique, l'acide méthanesulfonique ou l'acide benzènesulfonique.

7. Utilisation selon l'une quelconque des revendications 1 à 3, dans laquelle (B) est le maléate de (R)-5-[2-(5,6-diéthyl-indan-2-ylamino)-1-hydroxy-éthyl]-8-hydroxy-1H-quinoléin-2-one.

8. Utilisation selon l'une quelconque des revendications précédentes, dans laquelle le médicament contient en outre au moins une substance médicamenteuse qui est un anti-inflammatoire, un bronchodilatateur, un antihistaminique, un décongestionnant ou une substance médicamenteuse anti-tussive.

9. Utilisation selon l'une quelconque des revendications précédentes, dans laquelle le rapport molaire de (A) sur (B) est de 100:1 sur 1:300.

10. Utilisation selon la revendication 1, dans laquelle le médicament est une poudre sèche dans une capsule, la capsule contenant une dose unitaire de (A), une dose unitaire de (B) et un support pharmaceutiquement acceptable en une quantité pour amener le poids total de poudre sèche par capsule entre 5 mg et 50 mg.
